# EUROPÄISCHE PATENTSCHRIFT

(11) **EP 2 985 054 B1**
(45) Veröffentlichungstag und Bekanntmachung des Hinweises auf die Patenterteilung: **28.12.2016**
(21) Anmeldenummer: 15167595.6
(22) Anmeldetag: 13.05.2015
(51) Int. Cl.: A61N 1/08

(54) **IMPLANTIERBARES GERÄT MIT ELEKTRISCHEM FILTER**
IMPLANTABLE DEVICE WITH ELECTRICAL FILTER
APPAREIL IMPLANTABLE DOTÉ D'UN FILTRE ÉLECTRIQUE

(30) Priorität: 13.08.2014 US 201462036639 P
(43) Veröffentlichungstag der Anmeldung: 17.02.2016
(73) Patentinhaber: BIOTRONIK SE & Co. KG, 12359 Berlin (DE)
(72) Erfinder: Rump, Jens, 12049 Berlin (DE); Friedrich, Michael, 14532 Kleinmachnow (DE)
(74) Vertreter: Galander, Marcus

(56) Entgegenhaltungen:
- US-A1- 2009 149 906
- US-A1- 2010 099 281
- US-A1- 2011 172 756

## Beschreibung

Die Erfindung betrifft ein permanent oder temporär implantierbares Gerät mit einem proximalen und einem distalen Ende und mindestens einer dazwischen angeordneten, langgestreckten elektrisch leitfähigen Komponente sowie einem im Bereich des distalen Endes des Gerätes angeordneten Kontaktpol zum elektrischen Kontaktieren von im Betrieb an den Kontaktpol angrenzenden Körpergewebe, wobei der Kontaktpol mit der langgestreckten elektrisch leitfähigen Komponente elektrisch verbunden ist.

Solche Geräte, beispielsweise Elektrodenleitungen für die Elektrostimulation, haben den Nachteil, dass sich ihr elektrischer Leiter als langgestreckte, elektrisch leitfähige Komponente in einem Kernspintomografen erwärmen kann, weil die im Kernspintomografen herrschenden wechselnden Magnetfelder in dem elektrischen Leiter nicht unbeachtliche elektrische Ströme induzieren. Deshalb können Herzschrittmacherpatienten heutzutage in der Regel nicht oder nur eingeschränkt in einem Kernspintomografen untersucht werden.

An implantierbare Herzschrittmacher oder Defibrillatoren sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole als Kontaktpole zum Kontaktieren von Körpergewebe aufweist. Ein solcher Kontaktpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Zu diesen Zwecken bilden Kontaktpole typischerweise elektrisch leitende Oberflächenabschnitte einer Elektrodenleitung. Kontaktpole sind typischerweise als Ringelektrode in Form eines Rings um die Elektrodenleitung oder in Form einer Spitzen- oder Tipelektrode am distalen Ende der Elektrodenleitung vorgesehen. Die Kontaktpole sind über eine oder mehrere elektrische Leiter mit Anschlusskontakten des elektrischen Anschlusses der Elektrodenleitung an deren proximalem Ende elektrisch leitend verbunden. Die jeweiligen elektrischen Leiter bilden langgestreckte, elektrisch leitfähige Komponenten der Elektrodenleitung als implantierbarem Gerät. Somit verlaufen zwischen den Anschlusskontakten des elektrischen Anschlusses die Elektrodenleitungen an deren proximalen Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleitung bezeichnet. Solche Funktionsleitungen sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können. Außerdem besteht die Gefahr, dass äußere Felder zu signalverfälschenden Einstreuungen führen.

Die US 2009/149906 A1 offenbart Elektrodenleitungen zum Einsatz in Magnetresonanztomographen, die magneto-resistive Komponenten enthalten. Der Erfindung liegt die Aufgabe zugrunde, ein Gerät zu schaffen, welches einen Einsatz in einem Magnetresonanztomografen erlaubt.

Erfindungsgemäß wird diese Aufgabe durch ein Gerät gelöst, bei dem die langgestreckte elektrisch leitfähige Komponente eine mit der übrigen langgestreckten elektrisch leitfähigen Komponente in Serie geschaltete Teilkomponente enthält, die mindestens eine kapazitive Komponente, mindestens eine induktive Komponente und mindestens eine magneto-resistive Komponente enthält, die derart angeordnet und konfiguriert ist, dass eine Verringerung einer Resistivität der magneto-resistiven Komponente zu einer Verschiebung der Resonanzfrequenz der Teilkomponente führt.

Induktivitäten und dazu parallel geschaltete, z.B. parasitäre Kapazitäten der in Serie geschalteten Teilkomponente lassen sich derart gestalten, dass die in Serie geschaltete Teilkomponente hohe Impedanzen (> 1 kOhm) bei elektrischen Wellen im Radiofrequenzbereich aufweist. Die in Serie geschaltete Teilkomponente wirkt somit als Filterelement. Die magneto-resistive Komponente hat die Eigenschaft, in Anwesenheit eines Magnetfeldes einen geringeren Widerstand zu haben und kann daher die kapazitive und/oder die induktive Komponente der in Serie geschalteten Teilkomponente z.B. durch Kurzschluss so verändern, dass die Induktivität und/oder die Kapazität auf einem Abschnitt des Filterelementes infolge des Kurzschlusses in Anwesenheit von Magnetfeldern stark reduziert wird. Mit der geänderten Induktivität ändert sich die Resonanzfrequenz des Filterelementes und es erfolgt eine Abstimmung des Filters auf die Larmorfrequenz verschiedener Magnetresonaztomografen durch das Magnetfeld.

Erfindungsgemäß wird somit ein elektrisches Bauteil mit mehreren Komponenten vorgeschlagen, davon mindestens eine induktive, mindestens eine kapazitive und mindestens eine magneto-resistive Komponente, die so angeordnet und miteinander verschaltet sind, dass das elektrische Bauteil unterschiedliche Frequenzgänge aufweist, je nach dem ob das elektrische Bauteil einem Magnetfeld ausgesetzt ist.

Vorzugsweise enthält die magneto-resistive Komponente ein Material, dessen elektrischer Widerstand unter dem Einfluss eines Magnetfeldes abnimmt. Die magneto-resistive Komponente ist vorzugsweise so angeordnet, dass sie eine Lücke zwischen elektrisch leitenden Elementen oder eine Lücke in einem elektrisch leitenden Element der in Serie geschalteten Teilkomponente wenigstens teilweise überbrückt.

Die magneto-resistive Komponente weist vorzugsweise ein Material mit wenigstens einer der folgenden Eigenschaften auf: einen giant magnetoresistant (GMR) Effekt, einen anisotropic magnetoresistant (AMR) Effekt, einen colossal magnetoresistant (CMR) Effekt oder einen tunnel magnetoresistant (TMR) Effekt.

Gemäß einer Ausführungsvariante weist die magneto-resistive Komponente sich abwechselnde Schichten von ferromagnetischem und nicht-magnetischem Material auf Die magneto-resistive Komponente kann auf ein Substrat gedruckt sein.

Die induktive Komponente weist vorzugsweise einen spulenförmig auf eine geschlitzte Metallhülse gewickelten Draht und/oder eine spiralförmig auf eine geschlitzte Metallhülse gewickelte metallisierte Folie auf.

Die kapazitive Komponente weist vorzugsweise eine geschlitzte Doppelhülse auf.

Gemäß einer Ausführungsvariante sind die kapazitive, die induktive und die magneto-resistive Komponente der in Serie geschalteten Teilkomponente so angeordnet und verschaltet, dass eine Verringerung der Resistivität der magneto-resistiven Komponente zu einer verringerten Induktivität der induktiven Komponente führt.

Alternativ sind die kapazitive, die induktive und die magneto-resistive Komponente der in Serie geschalteten Teilkomponente so angeordnet und verschaltet, dass eine Verringerung der Resistivität der magneto-resistiven Komponente zu einer verringerten Kapazität der kapazitiven Komponente führt.

Gemäß einer weiteren Alternative sind die kapazitive, die induktive und die magneto-resistive Komponente der in Serie geschalteten Teilkomponente so angeordnet und verschaltet, dass eine Verringerung der Resistivität der magneto-resistiven Komponente sowohl zu einer verringerten Kapazität der kapazitiven Komponente als auch einer verringerten Induktivität der induktiven Komponente führt.

Gemäß einer vorteilhaften Ausführungsvariante sind die kapazitive, die induktive und die magneto-resistive Komponente der in Serie geschalteten Teilkomponente so angeordnet und verschaltet, dass eine Verringerung der Resistivität der magneto-resistiven Komponente zu einem mindestens teilweisen Kurzschluss der induktiven Komponente und damit zu einer Änderung der Induktivität führt.

Gemäß einer alternativen vorteilhaften Ausführungsvariante sind die kapazitive, die induktive und die magneto-resistive Komponente der in Serie geschalteten Teilkomponente so angeordnet und verschaltet, dass eine Verringerung der Resistivität der magneto-resistiven Komponente zu einem mindestens teilweisen Kurzschluss der kapazitiven Komponente und damit zu einer Änderung der Kapazität führt.

Gemäß einer alternativen vorteilhaften Ausführungsvariante sind die kapazitive, die induktive und die magneto-resistive Komponente der in Serie geschalteten Teilkomponente so angeordnet und verschaltet, dass eine Verringerung der Resistivität der magneto-resistiven Komponente zu einer weiteren Induktivität führt, die der Induktivität der induktiven Komponente entgegen wirkt.

Gemäß einer alternativen vorteilhaften Ausführungsvariante sind die kapazitive, die induktive und die magneto-resistive Komponente der in Serie geschalteten Teilkomponente so angeordnet und verschaltet, dass eine Verringerung der Resistivität der magneto-resistive Komponente zu einer Transformation der Induktivität der induktiven Komponente hin zu einer verringerten Induktivität führt.

Die letztgenannten vier Ausführungsvarianten können auch in verschiedenen Kombinationen miteinander kombiniert werden.

Die Erfindung soll nun anhand von Ausführungsbeispielen mit Bezug auf die Figuren näher erläutert werden. Die Figuren zeigen Folgendes:
- Fig. 1: zeigt als implantierbare medizinische Geräte einen implantierbaren Herzstimulator 10 und eine daran angeschlossene implantierbare Elektrodenleitung 20.
- Fig. 2: zeigt eine erste Variante einer erfindungsgemäßen Teilkomponente eines implantierbaren Gerätes;
- Fig. 3: zeigt eine zweite Variante einer erfindungsgemäßen Teilkomponente eines implantierbaren Gerätes;
- Fig. 4: zeigt eine dritte Variante einer erfindungsgemäßen Teilkomponente eines implantierbaren Gerätes;
- Fig. 5: zeigt eine vierte Variante einer erfindungsgemäßen Teilkomponente eines implantierbaren Gerätes;
- Fig. 6: zeigt eine fünfte Variante einer erfindungsgemäßen Teilkomponente eines implantierbaren Gerätes;
- Fig. 7: zeigt eine sechste Variante einer erfindungsgemäßen Teilkomponente eines implantierbaren Gerätes;
- Fig. 8: zeigt eine siebte Variante einer erfindungsgemäßen Teilkomponente eines implantierbaren Gerätes; und
- Fig. 9: zeigt eine achte Variante einer erfindungsgemäßen Teilkomponente eines implantierbaren Gerätes.

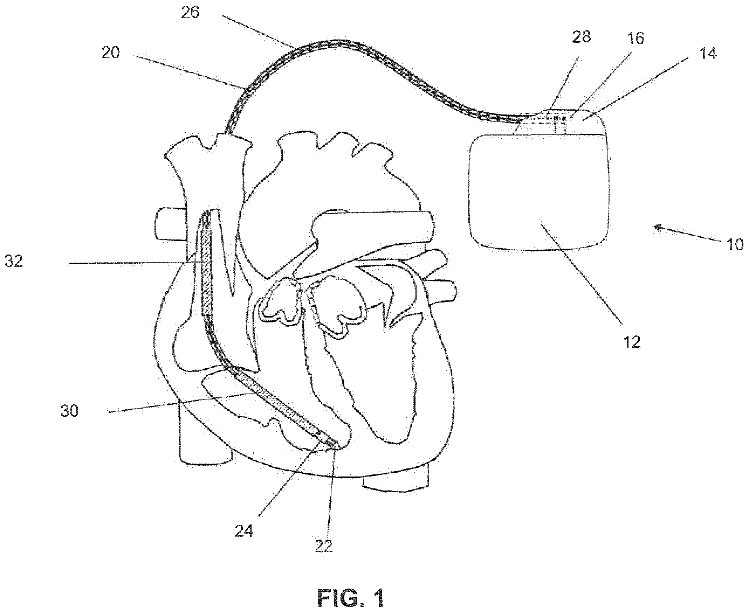

Figur 1 zeigt als implantierbare medizinische Geräte einen implantierbaren Herzstimulator 10 und eine daran angeschlossene implantierbare Elektrodenleitung 20.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tipelektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tipelektrode 22 und die Ringelektrode 24, im Anwendungsfall die Elektrodenleitung 20, im Apex eines rechten Ventrikels eines Herzens befinden.

Sowohl die Tipelektrode 22 als auch die Ringelektrode 24 sind über jeweils wenigstens einen elektrischen Leiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren. Die elektrischen Leiter 26 in der Elektrodenleitung 20 können als annähernd gestreckte Seilleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden werden im Rahmen dieses Textes als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.
Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist.

In dem Ausführungsbeispiel gemäß Figur 1 bildet die Elektrodenleitung 20 das implantierbare Gerät mit seinem proximalen und seinem distalen Ende. Der Leiter 26 der Elektrodenleitung 20 bildet eine zwischen dem proximalen und dem distalen Ende der Elektrodenleitung 20 angeordnete, langgestreckte elektrisch leitfähige Komponente. Die Elektrodenpole 22 und 24 bilden jeweils einen im Bereich des distalen Endes der Elektrodenleitung 20 angeordneten Kontaktpol zum elektrischen Kontaktieren von im Betrieb an den Kontaktpol angrenzenden Körpergewebe. Der Kontaktpol ist mit der langgestreckten elektrisch leitfähigen Komponente, nämlich dem Leiter 26, elektrisch verbunden.

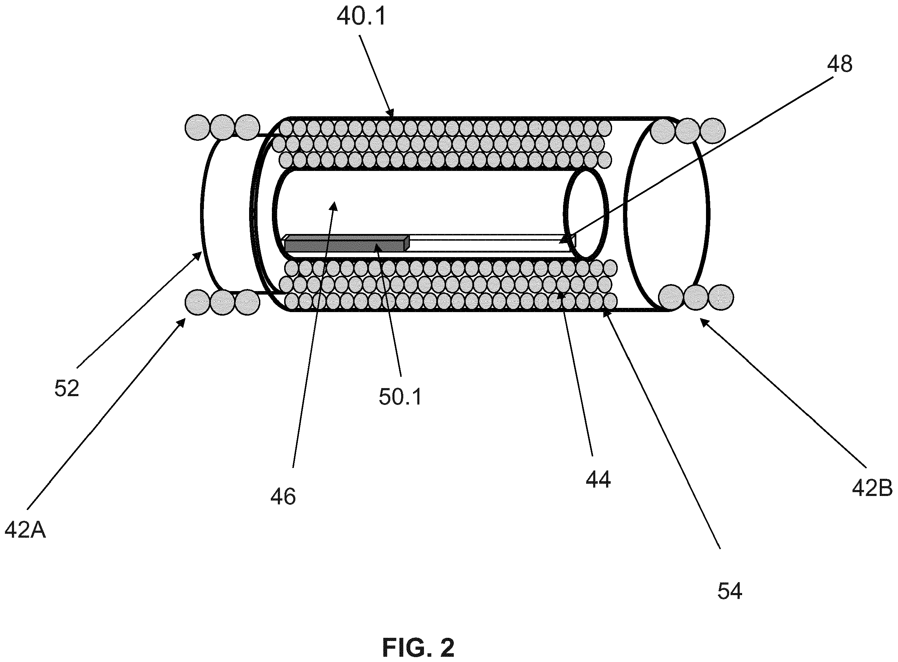

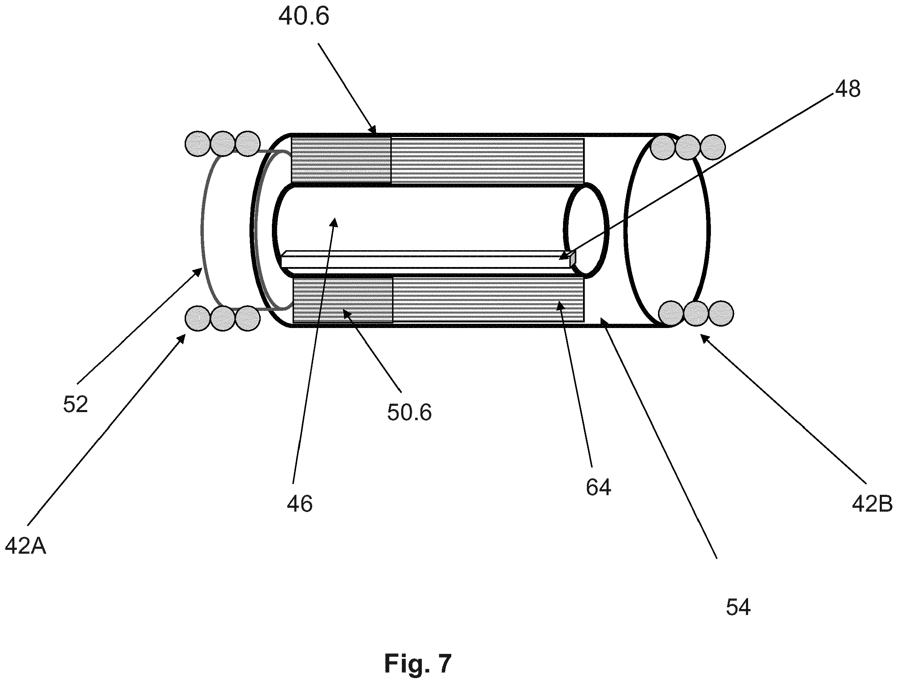

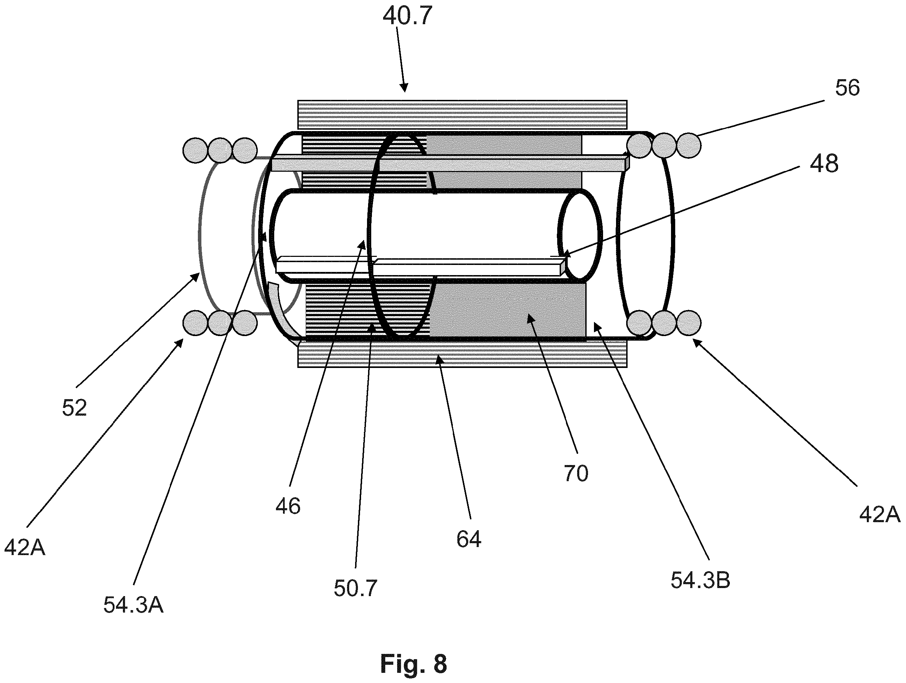

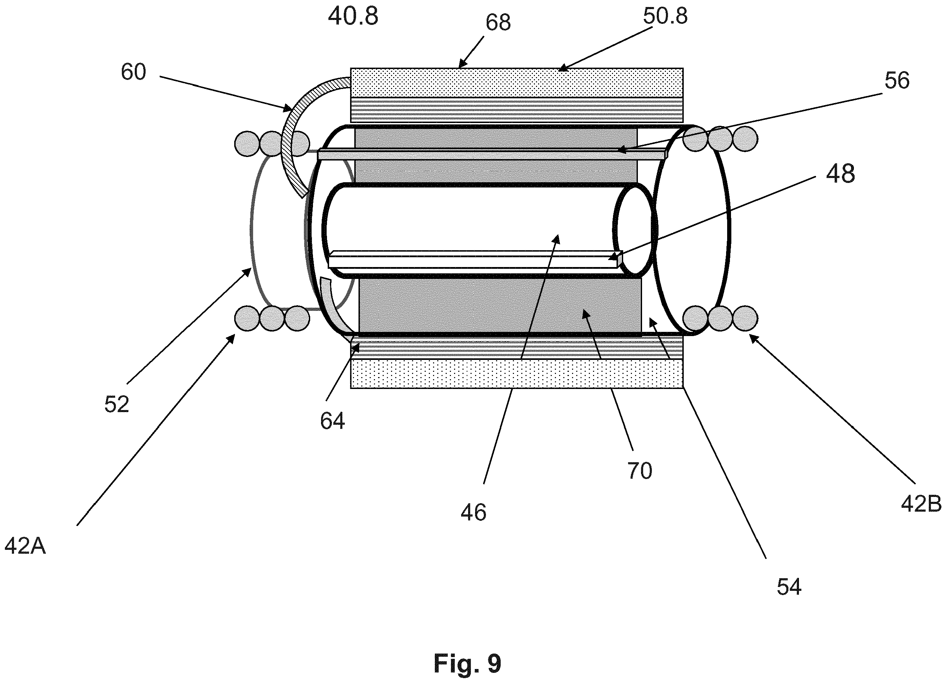

Figuren 2 bis 9 zeigen jeweils unterschiedliche Varianten einer Teilkomponente 40 der Elektrodenleitung 20, wobei die Teilkomponente 40 mit dem Leiter 26 der Elektrodenleitung 20 in Serie geschaltet ist.

Der Leiter 26 wird in den Ausführungsbeispielen wenigstens abschnittsweise von einer Drahtwendel 42 gebildet, die einen proximalen Abschnitt 42A und einen distalen Abschnitt 42B hat, zwischen denen die Teilkomponente 40 angeordnet ist.

Gemäß der in Fig. 2 dargestellten ersten Variante der Teilkomponente 40.1 weist die Teilkomponente 40.1 einen spulenförmig aufgewickelten Draht 44 als induktive Komponente auf. Die kapazitive Komponente ergibt sich aus den parasitären Kapazitäten zwischen den Drahtwicklungen des spulenförmig aufgewickelten Drahtes 44.

Der Draht 44 ist auf eine längsgeschlitzte, elektrisch leitende Metallhülse 46 aufgewickelt. Ein Längsschlitz 48 in der Metallhülse 46 ist wenigstens teilweise mit magneto-resistivem Material 50.1 aufgefüllt, welches die magneto-resistive Komponente bildet und den Schlitz 48 wenigstens auf einer Teillänge überbrückt. Die magneto-resistive Komponente 50.1 hat die Eigenschaft, bei Anwesenheit eines Magnetfeldes einen niedrigen elektrischen Widerstand zu haben, so dass die beiden Kanten der Metallhülse 46, die sich im Bereich des Schlitzes 48 gegenüberliegen und zwischen denen das magneto-resistive Material 50.1 angeordnet ist, über das magneto-resistive Material 50.1 elektrisch miteinander verbunden, also kurzgeschlossen sind. Liegt kein äußeres Magnetfeld an, ist das magneto-resistive Material 50.1 hochohmig und ein Stromfluss über den Schlitz 48 hinweg ist verhindert oder zumindest stark begrenzt.

Wenn das magneto-resistive Material 50.1 niederohmig ist, wirkt die geschlitzte Metallhülse 46 zumindest dort, wo der Schlitz 48 von dem magneto-resistiven Material 50.1 überbrückt ist, wie eine nicht-geschlitzte, geschlossene Metallhülse und damit wie eine kurzgeschlossene Spule mit einer einzigen Schlaufe. In diesem Fall wird die Induktivität der Windungen der äußeren Spule, die von dem aufgewickelten Draht 44 gebildet wird und die über der kurzgeschlossenen Metallhülse liegen, durch die kurzgeschlossene Metallhülse derart transformiert, dass die resultierende Induktivität nah oder gleich 0 Henry ist. Dies gilt für den Fall, dass das magneto-resistive Material 50.1 unter dem Einfluss eines äußeren Magnetfeldes einen niedrigen elektrischen Widerstand hat. Dann bricht die Induktivität der Teilkomponente 40.1 über der durch das magneto-resistive Material 50.1 kurzgeschlossenen Metallhülse 46 zusammen. Dadurch nimmt die gesamte Induktivität der Teilkomponente 40.1 stark ab und die Resonanzfrequenz der Teilkomponente 40.1 nimmt entsprechend zu.

Um die Teilkomponente 40.1 elektrisch mit der Drahtwendel 42 zu verbinden, ist außerdem noch eine elektrisch leitende Anschlaghülse 52 vorgesehen, die mit einer ebenfalls elektrisch leitenden Außenhülse 54 verbunden ist. Die von dem aufgewickelten Draht 44 gebildete äußere Spule sowie die geschlitzte Metallhülse 46 und das magneto-resistive Material 50.1 sind innerhalb der Außenhülse 54 angeordnet. Der distale Abschnitt 42B der Drahtwendel 42 ist elektrisch mit der Außenhülse 54 verbunden.

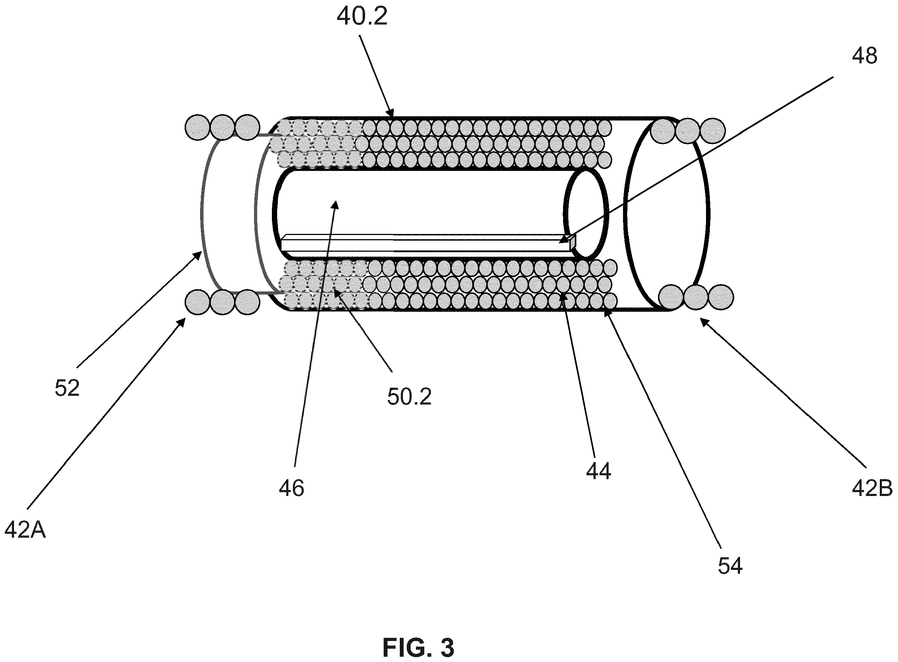

Der Aufbau der in Fig. 3 dargestellten zweiten Variante der Teilkomponente 40.2 ist in vielerlei Hinsicht ähnlich dem Aufbau der Teilkomponente 40.1 aus Fig. 2. Insbesondere sind wieder ein proximaler Abschnitt 42A und ein distaler Abschnitt 42B der als Leiter 26 dienenden Drahtwendel 42 zu erkennen, genauso wie die Anschlaghülse 52 und die Außenhülse 54. Wiederrum sind die induktive Komponente, die kapazitive Komponente und die magneto-resistive Komponente der Teilkomponente 40.2 innerhalb der Außenhülse 54 angeordnet.

Gemäß der zweiten Variante wird die induktive Komponente der Teilkomponente 40.2 wiederrum von einem spulenförmig aufgewickelten Draht 44 gebildet, der um eine geschlitzte Innenhülse 46 gewickelt ist. In diesem Fall kann die Innenhülse 46 aus Metall bestehen, muss aber keine Metallhülse sein. Falls die Innenhülse 46 keine Metallhülse ist, sondern aus nicht leitendem Material besteht, braucht sie auch keinen Schlitz 48 aufzuweisen.

Die kapazitive Komponente der Teilkomponente 40.2 ergibt sich auch hier wiederrum aus den parasitären Kapazitäten zwischen den Drahtwicklungen des spulenförmig aufgewickelten Drahtes 44.

Im Unterschied zu der in Fig. 2 dargestellten ersten Variante ist ein Teil des Drahtes bei der in Fig. 3 dargestellten zweiten Variante mit magneto-resistivem Material 50.2 beschichtet, d. h. das magneto-resistive Material 50.2, das die magneto-resistive Komponente bildet, befindet sich zwischen einzelnen Windungen des spulenförmig aufgewickelten Drahtes 44 und schließt diese Windungen miteinander kurz oder isoliert sie voneinander, je nachdem ob das magneto-resistive Material 50.2 einen niedrigen oder hohen elektrischen Widerstand hat. Wenn das magneto-resistive Material 50.2 in Anwesenheit eines Magnetfeldes einen niedrigen elektrischen Widerstand hat, ist derjenige Spulenabschnitt, in dem die Windungen des Drahtes 44 mit dem magneto-resistiven Material 50.2 beschichtet sind, kurzgeschlossen, so dass dieser Spulenabschnitt nicht mehr zur Gesamtinduktivität der Teilkomponente 40.2 beitragen kann. Falls kein äußeres Magnetfeld anliegt, so dass die magneto-resistive Komponente 50.2 einen hohen elektrischen Widerstand aufweist, ist die Gesamtinduktivität der Teilkomponenten 40.2 entsprechend höher.

Auch bei der zweiten Variante gemäß Fig. 3 nimmt die Resonanzfrequenz der Teilkomponente 40.2 bei Anwesenheit eines Magnetfeldes in Folge der dann geringeren Gesamtinduktivität entsprechend zu.

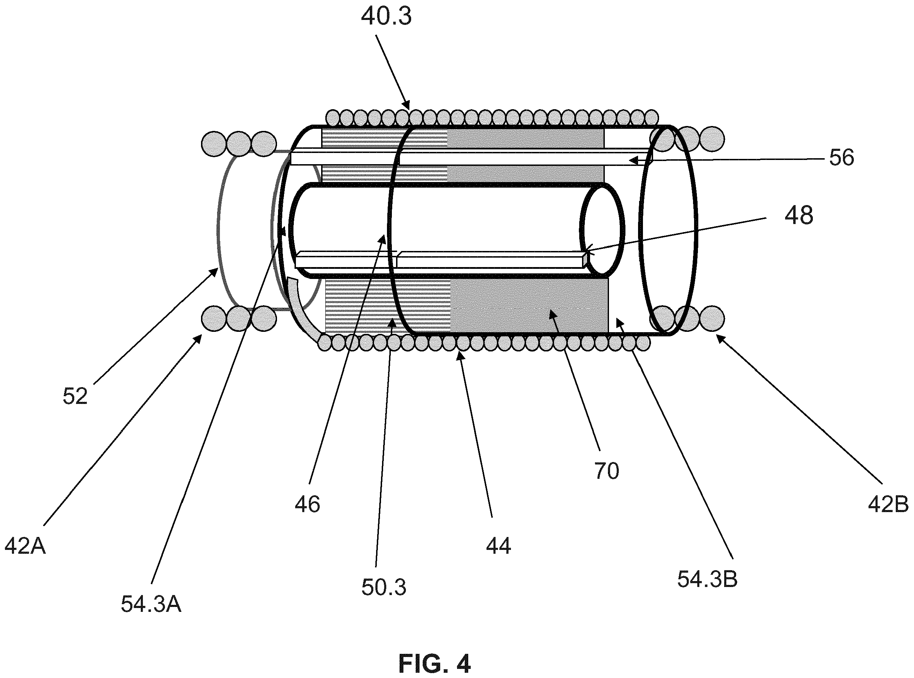

Auch bei der in Fig. 4 dargestellten dritten Variante der Teilkomponente 40.3 sind wie bei den ersten beiden Varianten eine Anschlaghülse 52 und eine Außenhülse 54 vorgesehen, die jeweils den proximalen Abschnitt 42A bzw. den distalen Abschnitt 42B der Drahtwendel 42 elektrisch kontaktieren.

Im Unterschied zu den zuerst beschriebenen beiden Varianten ist bei der in Fig. 4 abgebildeten dritten Variante jedoch die Außenhülse 54.3 geschlitzt und weist einen Schlitz 56 auf. Die Außenhülse 54.3 ist dabei eine längsgeschlitzte, elektrisch leitende Doppelmetallhülse. Die induktive Komponente der Teilkomponente 40.3 ergibt sich bei der dritten Variante aus einem spulenförmig aufgewickelten Draht 44, der in diesem Falle auf die längsgeschlitzte, elektrisch leitende Doppelmetallhülse 54.3 aufgewickelt ist.

Die kapazitive Komponente der Teilkomponente 40.3 wird von der elektrisch leitenden Doppelmetallhülse 54.3 gebildet, in dem im distalen Abschnitt 54.3B der Doppelmetallhülse zwischen dem inneren Hülsenteil 46 und dem äußeren Hülsenteil ein Dielektrikum 70 angeordnet ist, so dass innere und äußerer Hülsenteil der Doppelmetallhülse 54.3 kapazitiv wirken. Um eine hohe Kapazität zu erzielen, ist der Abstand zwischen innerem und äußerem Teil der Doppelmetallhülse 54.3 gering. Im proximalen Abschnitt 54.3A ist zwischen dem inneren und dem äußeren Teil der Doppelmetallhülse 54.3 magneto-resistives Material 50.3 vorgesehen.

Eine weitere kapazitive Komponente der Teilkomponente 40.3 ist durch die parasitären Kapazitäten zwischen den Drahtwicklungen des spulenförmig aufgewickelten Drahtes 44 gegeben, so dass sich die Gesamtkapazität der Teilkomponente 40.3 aus den parasitären Komponenten zwischen den Drahtwicklungen und den Kapazitäten zwischen dem inneren und äußeren Teilen der Doppelmetallhülse 54.3 ergibt.

Solange kein hinreichend starkes äußeres Magnetfeld anliegt, besitzt das magneto-resistive Material 50.3 zwischen dem inneren und dem äußeren Teil des proximalen Abschnitts 54.3A der Doppelmetallhülse 54.3 einen hohen elektrischen Widerstand und wirkt als Dielektrikum.

Falls ein hinreichend starkes äußeres Magnetfeld anliegt, besitzt das magneto-resistive Material 50.3 einen niedrigen elektrischen Widerstand und die Kapazität des proximalen Abschnitts 54.3A der Doppelmettallhülse 54.3 nimmt ab. Gleichzeitig nimmt auch die Induktivität der Teilkomponente 40.3 ab, weil die längsgeschlitzte, elektrisch leitende Doppelmetallhülse in ihrem proximalen Abschnitt 54.3A auch über den Schlitz hinweg kurzgeschlossen wird und somit als kurzgeschlossene Spule wirkt. Auf diese Weise sinkt auch die Induktivität der Teilkomponente 40.3 genau wie im Zusammenhang mit der ersten Variante in Fig. 2 beschrieben.

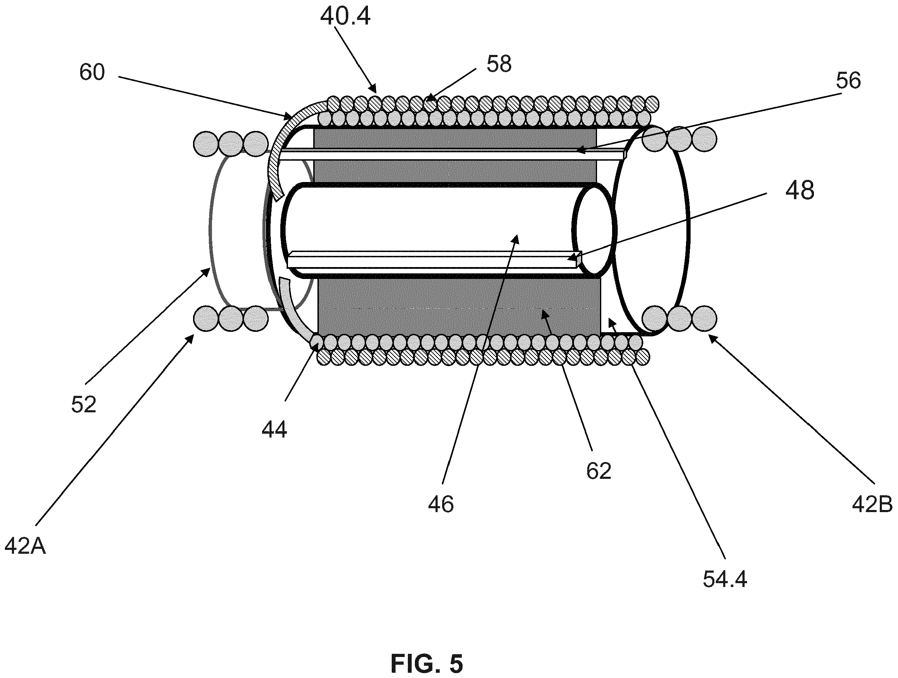

Bei der in Fig. 5 abgebildeten vierten Variante ist genau wie bei den zuvor beschriebenen Varianten eine Anschlaghülse 52 zum Kontaktieren eines proximalen Abschnitts 42A der Drahtwendel 42 vorgesehen sowie eine in diesem Falle wieder geschlitzte Außenhülse 54.4.

Bei der in Fig. 5 abgebildeten vierten Variante ist als induktive Komponente wiederrum ein spulenförmig aufgewickelter Draht 44 vorgesehen und die kapazitive Komponente der Teilkomponente 40.4 ergibt sich aus den parasitären Kapazitäten zwischen den Drahtwicklungen des Drahtes 44. Im Ausführungsbeispiel ist der Draht 44 auf die geschlitzte Außenhülse 54.4 aufgewickelt. Grundsätzlich könnte der Draht 44 auch auf eine in Fig. 5 ebenfalls abgebildete geschlitzte Innenhülse 46 aufgewickelt sein. Neben der von dem Draht 44 gebildeten ersten Spule 44 ist an der ebenfalls vom aufgewickelten Draht 58 gebildete zweite Spule mit gegenüber der ersten Spule entgegengesetztem Wicklungsinn vorgesehen. In dem dargestellten Ausführungsbeispiel ist die zweite Spule 58 oberhalb der ersten Spule 44 angeordnet, sie kann jedoch auch genauso gut unter dieser angeordnet sein. Während die von dem Draht 44 gebildete erste Spule dauerhaft mit der Drahtwendel 42 elektrisch verbunden ist, ist die von dem Draht 58 gebildete zweite Spule über ein magneto-resistives Koppelelement 60 mit der Drahtwendel 42 verbunden und somit nur dann wirksam, wenn das magneto-resistive Koppelelement 60 elektrisch leitend ist. Dies ist bei Anwesenheit entsprechend hoher Magnetfelder der Fall. In diesem Fall wird das magneto-resistive Koppelelement 60, welches die magneto-resistive Komponente der Teilkomponente 40.4 bildet, elektrisch leitend und die vom Draht 58 gebildete zweite Spule wirksam. Wegen des entgegengesetzten Wicklungssinn der zweiten Spule heben sich die Magnetfelder der von dem Draht 44 gebildeten ersten Spule und der von dem Draht 58 gebildeten zweiten Spule wenigstens annähernd auf, wenn das magneto-resistive Koppelelement 60 unter dem Einfluss eines entsprechend starken Magnetfeldes leitend wird, so dass die Gesamtinduktivität der Teilkomponente 40.4 in diesem Falle abnimmt.

In dem in Fig. 5 dargestellten Ausführungsbeispiel der vierten Variante ist zwischen der geschlitzten Innenhülse 46 und der geschlitzten Außenhülse 54.4 noch eine dielektrische Schicht 62 als Dielektrikum vorgesehen, so dass die geschlitzte Innenhülse 46 und die geschlitzte Außenhülse 54.4 zusätzlich auch noch eine Kapazität bilden und eine weitere kapazitive Komponente der Teilkomponente 40.4 darstellen.

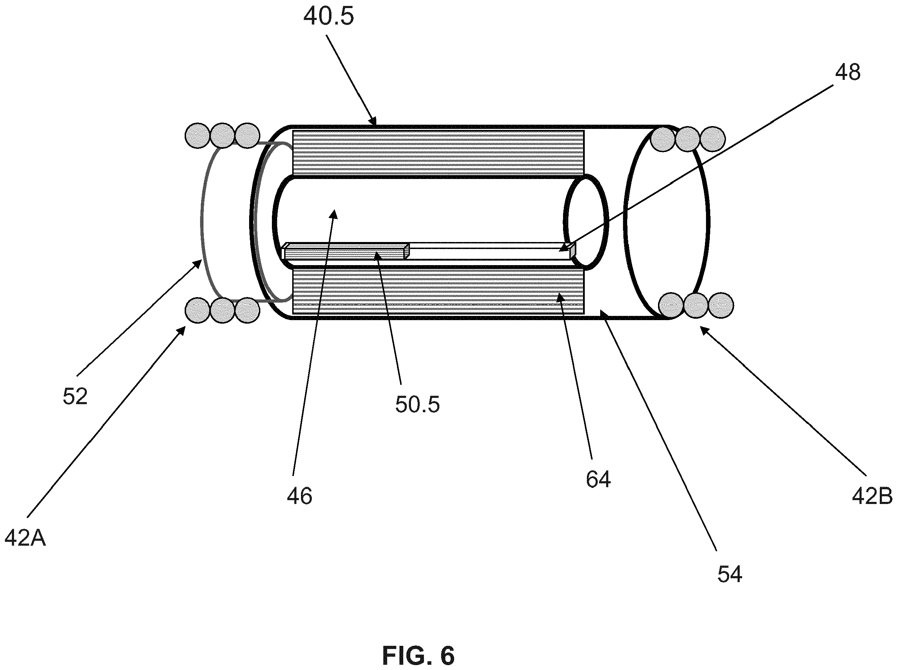

Die in den Fig. 6 bis 9 dargestellten Varianten 40.5, 40.6, 40.7 und 40.8 unterscheiden sich von den in den Fig. 2 bis 5 dargestellten Varianten dadurch, dass bei den Varianten gemäß der Fig. 6 bis 9 die induktive Komponente jeweils von einer spiralförmig gewickelten metallisierten Folie 64 und - im Falle der achten Variante, die der vierten Variante entspricht - der Folie 68 gebildet ist. Je nach Ausführungsvariante ist die metallisierte Folie 64 (wie in den entsprechenden Varianten der Draht 44) mit magneto-resistivem Material 50.8 beschichtet. Die magneto-resistiven Materialien 50 sind in den Figuren 6 bis 9 mit 50.5, 50.6, 50.7 und 50.8 gekennzeichnet.
Für alle Varianten gilt, dass die Teilkomponente 40 an einer beliebigen Stelle entlang des Innenleiters 26 einer Elektrodenleitung 20 platziert sein kann.
Ebenso kann die jeweilige Teilkomponente 40 an einer beliebigen Stelle entlang des Außenleiters einer Elektrodenleitung 20 platziert sein.
Bei als Seilen ausgeführten elektrischen Leitern 26 kann die Teilkomponente 40 ebenfalls an einer beliebigen Stelle entlang des Außenleiters einer Elektrodenleitung 20 platziert sein.

Da die meisten magneto-resistiven Effekte anisotrop, also abhängig von der Ausrichtung im Magnetfeld sind, könnte die magneto-resistive Komponente geometrisch so gestaltet werden, dass sie dennoch isotrop - oder zumindest in jeder Feldausrichtung ausreichend - reagiert. Zum Beispiel könnten drei orthogonal ausgerichtete Elemente elektrisch parallel geschaltet werden. Bei der zweiten bis vierten Variante ist die Feldempfindlichkeit aufgrund der Rotationssymmetrie bereits in zwei Richtungen gleich, und durch geschickte Wahl der axialen Ausdehnung des magneto-resistiven Elementes kann eine Isotropie hergestellt werden.

Auch ist die Selbstjustage des durch die Teilkomponente gebildeten Filters für mehr als zwei Magnetfeldstärken, beispielsweise für die drei Stärken 1,5, 3 und 7 T durch den Einsatz von mehreren Elementen, die bei verschiedener Feldstärke wirksam werden, machbar.

Die Teilkomponente ist vorzugsweise ein Filter, der als Bandsperre wirkt und der bei verschiedenen Larmorfrequenzen, insbesondere für 1,5T- und 3T-Geräte, eine Wirkung > 1 kOhm aufweist. Die Resonanzfrequenz des Bandstoppfilters verschiebt sich je nach Amplitude des magnetischen Feldes derart, dass die Sperrfrequenz jeweils mit der Larmorfrequenz übereinstimmt.

Die in Serie geschaltete Teilkomponente 40 hat, wie vorstehend dargelegt, vorzugsweise eine oder mehrere der folgenden Eigenschaften:
- die magneto-resistive Komponente weist einen giant magnetoresistant (GMR) Effekt auf
- die magneto-resistive Komponente weist einen anisotropic magnetoresistant (AMR) Effekt auf
- die magneto-resistive Komponente weist einen colossal magnetoresistant (CMR) Effekt auf
- die magneto-resistive Komponente weist einen tunnel magnetoresistant (TMR) Effekt auf
- die magneto-resistive Komponente weist sich abwechselnde Schichten von ferromagnetischem und nicht-magnetischem Material auf
- die magneto-resistive Komponente ist auf ein Substrat gedruckt
- die induktive Komponente weist einen spulenförmig auf eine geschlitzte Metallhülse gewickelten Draht auf
- die induktive Komponente weist eine spiralförmig auf eine geschlitzte Metallhülse gewickelte metallisierte Folie auf
- die kapazitive Komponente weist eine geschlitzte Doppelhülse auf
- eine Verringerung der Resistivität der magneto-resistiven Komponente führt zu einer verringerten Induktivität der induktiven Komponente.
- eine Verringerung der Resistivität der Magneto-resistiven Komponente führt zu einer verringerten Kapazität der kapazitiven Komponente.
- eine Verringerung der Resistivität der magneto-resistiven Komponente führt sowohl zu einer verringerten Kapazität der kapazitiven Komponente als auch einer verringerten Induktivität der induktiven Komponente.
- eine Verringerung der Resistivität der magneto-resistiven Komponente führt zu einem mindestens teilweisen Kurzschluss der induktiven Komponente.
- eine Verringerung der Resistivität der magneto-resistiven Komponente führt zu einem mindestens teilweisen Kurzschluss der kapazitiven Komponente.
- eine Verringerung der Resistivität der magneto-resistiven Komponente führt zu einer Induktivität, die der Induktivität der induktiven Komponente entgegen wirkt.
- eine Verringerung der Resistivität der magneto-resistiven Komponente führt zu einer Transformation der Induktivität der induktiven Komponente hin zu einer verringerten Induktivität.

Durch die erfindungsgemäße Teilkomponente ist ein Bandstoppfilter realisierbar, dessen Wirkungsbereich auf einen großen Frequenzbereich ausgeweitet ist, ohne dass prinzipielle Änderungen der Bauart des Filters notwendig werden. Es sind keine zusätzlichen expliziten Bauteile nötig.

### Bezugszeichenliste

- 10: Herzstimulator
- 12: Gehäuse
- 14: Anschlussgehäuse
- 16: elektrische Kontakte
- 20: Elektrodenleitung
- 22: Elektrodenpol/Spitzen- oder Tipelektrode
- 24: Elektrodenpol/Ringelektrode
- 26: elektrischer Leiter/Innenleiter
- 28: Stecker/Steckkontakt
- 30: Elektrodenpol
- 32: Elektrodenpol
- 40: Teilkomponente
- 42: Drahtwendel
- 42A: proximaler Abschnitt
- 42B: distaler Abschnitt
- 44: spulenförmig aufgewickelter Draht/Spule
- 46: Metallhülse/Innenhülse
- 48: Längsschlitz
- 50: magneto-resistive Komponente/Material
- 52: Anschlaghülse
- 54: Außenhülse
- 56: Schlitz
- 58: Draht/zweite Spule
- 60: magneto-resistives Koppelelement
- 62: dielektrische Schicht
- 64: metallisierte Folie
- 68: Folie
- 70: Dielektrikum

## Patentansprüche

1. Implantierbares Gerät (20) mit einem proximalen und einem distalen Ende und mindestens einer dazwischen angeordneten, langgestreckten elektrisch leitfähigen Komponente (26) und einem im Bereich des distalen Endes des Gerätes angeordneten Kontaktpol (22, 24) zum elektrischen Kontaktieren von im Betrieb an den Kontaktpol (22, 24) angrenzenden Körpergewebe, wobei der Kontaktpol (22, 24) mit der langgestreckten elektrisch leitfähigen Komponente (26) elektrisch verbunden ist,
**dadurch gekennzeichnet, dass** die langgestreckte elektrisch leitfähige Komponente (26) eine mit der übrigen langgestreckten elektrisch leitfähigen Komponente in Serie geschaltete Teilkomponente (40) enthält, die mindestens eine kapazitive Komponente, mindestens eine induktive Komponente und mindestens eine magneto-resistive Komponente (50) enthält, die derart angeordnet und konfiguriert sind, dass eine Verringerung einer Resistivität der magneto-resistiven Komponente (50) zu einer Verschiebung der Resonanzfrequenz der Teilkomponente (40) führt.

2. Implantierbares Gerät gemäß Anspruch 1, bei dem die magneto-resistive Komponente (50) ein Material aufweist, dessen elektrischer Widerstand unter dem Einfluss eines Magnetfeldes abnimmt.

3. Implantierbares Gerät gemäß Anspruch 1 oder 2, bei dem die magneto-resistive Komponente (50) so angeordnet ist, dass sie eine Lücke zwischen elektrisch leitenden Elementen (40.4, 52) oder eine Lücke in einem elektrisch leitenden Element (46) der in Serie geschalteten Teilkomponente (40) wenigstens teilweise überbrückt.

4. Implantierbares Gerät gemäß einem der Ansprüche 1 bis 3, bei dem die magneto-resistive Komponente (50) ein Material mit wenigstens einer der folgenden Eigenschaften aufweist: einen giant magnetoresistant (GMR) Effekt, einen anisotropic magnetoresistant (AMR) Effekt, einen colossal magnetoresistant (CMR) Effekt oder einen tunnel magnetoresistant (TMR) Effekt.

5. Implantierbares Gerät gemäß einem der Ansprüche 1 bis 4, bei dem die magneto-resistive Komponente (50) sich abwechselnde Schichten von ferromagnetischem und nicht-magnetischem Material aufweist.

6. Implantierbares Gerät gemäß einem der Ansprüche 1 bis 5, bei dem die magneto-resistive Komponente (50) auf ein Substrat gedruckt ist.

7. Implantierbares Gerät gemäß einem der Ansprüche 1 bis 6, bei dem die induktive Komponente vorzugsweise einen spulenförmig auf eine geschlitzte Metallhülse (46) gewickelten Draht (44) und/oder eine spiralförmig auf eine geschlitzte Metallhülse (46) gewickelte metallisierte Folie (64) aufweist.

8. Implantierbares Gerät gemäß einem der Ansprüche 1 bis 7, bei dem die kapazitive Komponente eine geschlitzte Doppelhülse (54.3) aufweist.

9. Implantierbares Gerät gemäß einem der Ansprüche 1 bis 8, bei dem die kapazitive, die induktive und die magneto-resistive Komponente (50) der in Serie geschalteten Teilkomponente (40) so angeordnet und verschaltet sind, dass eine Verringerung der Resistivität der magneto-resistiven Komponente (50) zu einer verringerten Induktivität der induktiven Komponente führt.

10. Implantierbares Gerät gemäß einem der Ansprüche 1 bis 9, bei dem die kapazitive, die induktive und die magneto-resistive Komponente (50) der in Serie geschalteten Teilkomponente (40) so angeordnet und verschaltet sind, dass eine Verringerung der Resistivität der magneto-resistiven Komponente (50) zu einer verringerten Kapazität der kapazitiven Komponente führt.

11. Implantierbares Gerät gemäß einem der Ansprüche 1 bis 10, bei dem die kapazitive, die induktive und die magneto-resistive Komponente (50) der in Serie geschalteten Teilkomponente (40) so angeordnet und verschaltet sind, dass eine Verringerung der Resistivität der magneto-resistiven Komponente (50) sowohl zu einer verringerten Kapazität der kapazitiven Komponente als auch einer verringerten Induktivität der induktiven Komponente führt.

12. Implantierbares Gerät gemäß einem der Ansprüche 1 bis 11, bei dem die kapazitive, die induktive und die magneto-resistive Komponente (50) der in Serie geschalteten Teilkomponente (40) so angeordnet und verschaltet sind, dass eine Verringerung der Resistivität der magneto-resistiven Komponente (50) zu einem mindestens teilweisen Kurzschluss der induktiven Komponente und damit zu einer Änderung der Induktivität führt.

13. Implantierbares Gerät gemäß einem der Ansprüche 1 bis 12, bei dem die kapazitive, die induktive und die magneto-resistive Komponente (50) der in Serie geschalteten Teilkomponente (40) so angeordnet und verschaltet sind, dass eine Verringerung der Resistivität der magneto-resistiven Komponente (50) zu einem mindestens teilweisen Kurzschluss der kapazitiven Komponente und damit zu einer Änderung der Kapazität führt.

14. Implantierbares Gerät gemäß einem der Ansprüche 1 bis 13, bei dem die kapazitive, die induktive und die magneto-resistive Komponente (50) der in Serie geschalteten Teilkomponente 40 so angeordnet und verschaltet sind, dass eine Verringerung der Resistivität der magneto-resistiven Komponente (50) zu einer weiteren Induktivität führt, die der Induktivität der induktiven Komponente entgegen wirkt.

15. Implantierbares Gerät gemäß einem der Ansprüche 1 bis 14, bei dem die kapazitive, die induktive und die magneto-resistive Komponente (50) der in Serie geschalteten Teilkomponente (40) so angeordnet und verschaltet sind, dass eine Verringerung der Resistivität der magneto-resistiven Komponente (50) zu einer Transformation der Induktivität der induktiven Komponente hin zu einer verringerten Induktivität führt.

## Claims

1. An implantable device (20) having a proximal and a distal end and at least one elongate, electrically conductive component (26) arranged therebetween and a contact pole (22, 24) arranged in the region of the distal end of the device for electrically contacting bodily tissue adjacent to the contact pole (22, 24) during operation, wherein the contact pole (22, 24) is electrically connected to the elongate electrically conductive component (26),
**characterised in that** the elongate electrically conductive component (26) contains a sub-component (40), which is connected in series with the remaining elongate electrically conductive component and which contains at least one capacitive component, at least one inductive component and at least one magneto-resistive component (50), which are arranged and configured in such a way that a reduction of a resistivity of the magneto-resistive component (50) leads to a shift of the resonance frequency of the sub-component (40).

2. The implantable device as claimed in Claim 1, wherein the magneto-resistive component (50) comprises a material of which the electrical resistance decreases under the influence of a magnetic field.

3. The implantable device as claimed in Claim 1 or 2, wherein the magneto-resistive component (50) is arranged such that it at least partially bridges a gap between electrically conductive elements (40.4, 52) or a gap in an electrically conductive element (46) of the sub-component (40) connected in series.

4. The implantable device as claimed in any one of Claims 1 to 3, wherein the magneto-resistive component (50) comprises a material having at least one of the following properties: a giant magnetoresistance (GMR) effect, an anisotropic magnetoresistance (AMR) effect, a colossal magnetoresistance (CMR) effect or a tunnel magnetoresistance (TMR) effect.

5. The implantable device as claimed in any one of Claims 1 to 4, wherein the magneto-resistive component (50) has alternating layers of ferromagnetic and nonmagnetic material.

6. The implantable device as claimed in any one of Claims 1 to 5, wherein the magneto-resistive component (50) is printed onto a substrate.

7. The implantable device as claimed in any one of Claims 1 to 6, wherein the inductive component preferably has a wire (44) wound in a coiled manner on a slitted metal sleeve (46) and/or a metallised film (64) wound in a spiralled manner on a slitted metal sleeve (46).

8. The implantable device as claimed in any one of Claims 1 to 7, wherein the capacitive component has a slitted double sleeve (54.3).

9. The implantable device as claimed in any one of Claims 1 to 8, wherein the capacitive, the inductive and the magneto-resistive component (50) of the sub-component (40) connected in series are arranged and connected such that a reduction of the resistivity of the magneto-resistive component (50) leads to a reduced inductance of the inductive component.

10. The implantable device as claimed in any one of Claims 1 to 9, wherein the capacitive, the inductive and the magneto-resistive component (50) of the sub-component (40) connected in series are arranged and connected such that a reduction of the resistivity of the magneto-resistive component (50) leads to a reduced capacitance of the capacitive component.

11. The implantable device as claimed in any one of Claims 1 to 10, wherein the capacitive, the inductive and the magneto-resistive component (50) of the sub-component (40) connected in series are arranged and connected such that a reduction of the resistivity of the magneto-resistive component (50) leads both to a reduced capacitance of the capacitive component and to a reduced inductance of the inductive component.

12. The implantable device as claimed in any one of Claims 1 to 11, wherein the capacitive, the inductive and the magneto-resistive component (50) of the sub-component (40) connected in series are arranged and connected such that a reduction of the resistivity of the magneto-resistive component (50) leads to an at least partial short circuit of the inductive component and therefore to a change of the inductance.

13. The implantable device as claimed in any one of Claims 1 to 12, wherein the capacitive, the inductive and the magneto-resistive component (50) of the sub-component (40) connected in series are arranged and connected such that a reduction of the resistivity of the magneto-resistive component (50) leads to an at least partial short circuit of the capacitive component and therefore to a change of the capacitance.

14. The implantable device as claimed in any one of Claims 1 to 13, wherein the capacitive, the inductive and the magneto-resistive component (50) of the sub-component (40) connected in series are arranged and connected such that a reduction of the resistivity of the magneto-resistive component (50) leads to a further inductance that counteracts the inductance of the inductive component.

15. The implantable device as claimed in any one of Claims 1 to 14, wherein the capacitive, the inductive and the magneto-resistive component (50) of the sub-component (40) connected in series are arranged and connected such that a reduction of the resistivity of the magneto-resistive component (50) leads to a transformation of the inductance of the inductive component toward a reduced inductance.

## Revendications

1. Appareil implantable (20) avec une extrémité proximale et une extrémité distale et, disposé entre elles, au moins un composant électriquement conducteur (26) allongé et un pôle de contact (22, 24) disposé dans la région de l'extrémité distale de l'appareil pour la mise en contact électrique de tissus du corps adjacents au pôle de contact (22, 24) lors du fonctionnement, où le pôle de contact (22, 24) est relié électriquement avec le composant électriquement conducteur (26) allongé, **caractérisé en ce que** le composant électriquement conducteur (26) allongé contient un composant partiel (40), branché en série avec les composants électriquement conducteurs allongés restants, qui contient au moins un composant capacitif, au moins un composant inductif et au moins un composant magnéto-résistif, qui sont agencés et configurés de telle manière qu'une diminution de la résistivité du composant magnéto-résistif (50) provoque un décalage de la fréquence de résonance du composant partiel (40).

2. Appareil implantable selon la revendication 1, dans lequel le composant magnéto-résistif (50) présente un matériau dont la résistance électrique diminue sous l'influence d'un champ magnétique.

3. Appareil implantable selon la revendication 1, ou 2, dans lequel le composant magnéto-résistif (50) est disposé de manière à ce qu'il enjambe au moins partiellement une lacune entre des éléments (40.4, 52) électriquement conducteurs ou une lacune dans un élément (46) électriquement conducteur des composants partiels (40) branchés en série.

4. Appareil implantable selon l'une des revendications 1 à 3, dans lequel le composant magnéto-résistif (50) présente un matériau doté au moins de l'une des propriétés suivantes : un effet de magnétorésistance géante (GMR), un effet de magnétorésistance anisotrope (AMR), un effet de magnétorésistance colossale (CMR) ou un effet de magnétorésistance tunnel (TMR).

5. Appareil implantable selon l'une des revendications 1 à 4, dans lequel le composant magnéto-résistif (50) présente des couches alternées de matériaux ferromagnétique et non ferromagnétique.

6. Appareil implantable selon l'une des revendications 1 à 5, dans lequel le composant magnéto-résistif (50) est imprimé sur un substrat.

7. Appareil implantable selon l'une des revendications 1 à 6, dans lequel le composant inductif présente de préférence un fil (44) enroulé en forme de bobine sur une douille métallique (46) et/ou un film (64) métallisé enroulé en forme de spirale sur une douille métallique (46).

8. Appareil implantable selon l'une des revendications 1 à 7, dans lequel le composant capacitif présente une douille double (54.3) fendue.

9. Appareil implantable selon l'une des revendications 1 à 8, dans lequel les composants capacitif, inductif et magnéto-résistif (50) sont agencés et connectés au composant partiel (40) branché en série de telle manière qu'une diminution de la résistivité du composant magnéto-résistif (50) provoque une inductance diminuée dans le composant inductif.

10. Appareil implantable selon l'une des revendications 1 à 9, dans lequel les composants capacitif, inductif et magnéto-résistif (50) sont agencés et connectés au composant partiel (40) branché en série de telle manière qu'une diminution de la résistivité du composant magnéto-résistif (50) provoque une capacité diminuée dans le composant capacitif.

11. Appareil implantable selon l'une des revendications 1 à 10, dans lequel les composants capacitif, inductif et magnéto-résistif (50) sont agencés et connectés au composant partiel (40) branché en série de telle manière qu'une diminution de la résistivité du composant magnéto-résistif (50) provoque à la fois une capacité du composant capacitif diminuée ainsi qu'une inductance diminuée dans le composant inductif.

12. Appareil implantable selon l'une des revendications 1 à 11, dans lequel les composants capacitif, inductif et magnéto-résistif (50) sont agencés et connectés au composant partiel (40) branché en série de telle manière qu'une diminution de la résistivité du composant magnéto-résistif (50) provoque au moins un court-circuit partiel du composant inductif et par conséquent, une modification d'inductance.

13. Appareil implantable selon l'une des revendications 1 à 12, dans lequel les composants capacitif, inductif et magnéto-résistif (50) sont agencés et connectés au composant partiel (40) branché en série de telle manière qu'une diminution de la résistivité du composant magnéto-résistif (50) provoque au moins un court-circuit partiel du composant capacitif et par conséquent, une modification de la capacité.

14. Appareil implantable selon l'une des revendications 1 à 13, dans lequel les composants capacitif, inductif et magnéto-résistif (50) sont agencés et connectés au composant partiel (40) branché en série de telle manière qu'une diminution de la résistivité du composant magnéto-résistif (50) provoque une nouvelle inductance qui agit contre l'inductance du composant inductif.

15. Appareil implantable selon l'une des revendications 1 à 14, dans lequel les composants capacitif, inductif et magnéto-résistif (50) sont agencés et connectés au composant partiel (40) branché en série de telle manière qu'une diminution de la résistivité du composant magnéto-résistif (50) provoque une transformation de l'inductance du composant inductif vers une inductance plus faible.
